# EUROPEAN PATENT APPLICATION

(11) **EP 3 293 253 A1**
(43) Date of publication of application: **14.03.2018**
(21) Application number: 16188514.0
(22) Date of filing: 13.09.2016
(51) Int. Cl.: C12M 1/12, B01J 20/10, C03C 1/00, C12N 5/00, C12M 3/00

(54) **PROCESS FOR PRODUCING A GLASS MACROPOROUS MATRIX AND USES**

(71) Applicant: Carroucell, 73800 Saint-Helene-du-Lac (FR)
(72) Inventor: FATHALLAH, Tarek, 38500 VOIRON (FR); FERGUSON, Michael, 38240 MEYLAN (FR)
(74) Representative: Novagraaf Technologies

(57) **Abstract**

The invention relates to a process for producing a glass macroporous matrix, comprising the steps of :
1) filing a mold with glass microparticles,
2) filing the mold obtained in step 1) with a sol-gel solution comprising a compound selected among silicon dioxide, bore oxide, aluminium oxide and titanium oxide, thereby covering the glass microparticles,
3) gelling and drying at room temperature the sol-gel solution, thereby obtaining a glass material containing the glass microparticles, and
4) eventually removing the porous glass material containing the glass microparticles from the mold,
thereby obtaining the glass macroporous matrix.

The invention also relates to a process of cell culturing comprising the steps of:
1) preparing a glass macroporous matrix by the process as defined above,
2) applying cells at the surface of the matrix,
3) culturing the cells under culture conditions allowing the cells to colonize the matrix.

## Description

### Technical field

The present invention refers to processes for producing a glass macroporous matrix, and to a process of cell culturing.

Therefore, the present invention has utility especially in the field of cell or tissue culture.

In the description below, the references into brackets **([])** refer to the listing of references situated at the end of the text.

### Background

Two-dimensional plastic dishes have been used as a standard device for cell or tissue culture for several decades. However, such two-dimensional plastic dishes cannot mimic natural extracellular matrices environments. Compared to two-dimensional cell culture systems, the cellular activities and cell shapes observed in three-dimensional cell culture systems are more similar to the results from in vivo. Three-dimensional bioreactors offer a highly scalable technology that incorporates the necessary degree of control over the cell culture environment. Under agitation, their volumes can range from milliliters, to tens of liters, or more. Therefore, especially due to the recent developments in the life sciences such as regenerative medicine and pharmaceutical research, there is an increasing demand for cell culture devices with three-dimensional structures.

Microspheres may be used as microcarriers, offering a support that is capable of supporting the expansion, and potentially the subsequent differentiation, of stem cells in a 3- dimensional environment. They have been proven successful for the culture of human embryonic stem cells, starting from single cell seeding, in a defined and scalable environment under agitation.

Document WO2014197845 **([1])** describes devices for cell or tissue culture, comprising a three-dimensional structure, which further includes fibrils with beads and/or particles, and a method for manufacturing this device comprising the steps of (1) preparing a polymer solution which comprises beads, (2) spinning the polymer solution to form fibrils with the beads, and (3) using the fibrils with the beads to form the device for cell or tissue culture. However, this device needs to be hydrated before using. Therefore, size and shape of the material may differ considerably initial ones, which causes difficulties in sizing the device. Furthermore, the mechanical strength of the material is weak. Consequently, this device is not suitable for regeneration of hard tissue such as bone material.

Besides, recent processes allow obtaining complexe 3D shapes by sol-gel process.

For example, SIVARA® process (DEGUSSA NOVARA) allows implementing optical quality objects by sol-gel process following a supercritic drying, followed by a calcination at 1500°C.

Document WO201345671 **([2])** descrbibes a process for manufacturing an object made of a constituent material obtained from a sol-gel solution, successively comprising the following steps: a) a step of introducing said sol-gel solution into a mould of the object to be manufactured; b) a step of gelling the sol-gel solution; c) a step of drying said gel obtained in b) in said mould, by means of which said gel is converted into the constituent material of the aforementioned object. This process allows implementing 3D xerogel monoliths having complexe structures.

However, the difficulty to shape 3D the sol-gel matrices imply that sol-gel are are mainly used in thin layer.

Thus, a need exists of alternative processes to prepare sol-gel matrices, easier to implement and allowing a great control of the features of sol-gel matrice.

### Description of the invention

The present invention fulfills these and other needs.

The invention allows implementing macroporous 3D sol-gel matrices. The porosity of the matrices may vary from a few micrometers to hundreds of micrometers.

The size and the shape of the porosity of the matrix may be controled by choosing or controlling the physical features of the process of the invention.

The matrices prepared by processes of the invention can be used for culturing, analysing, studying the cells and for tissue regeneration.

Accordingly, in a first aspect, the present invention provides a process for producing a glass macroporous matrix, comprising the steps of:
1) filing a mold with glass microparticles,
2) filing the mold obtained in step 1) with a sol-gel solution comprising silicon dioxide, thereby covering the glass microparticles,
3) gelling and drying at rool temperature the sol-gel solution, thereby obtaining a glass material containing the glass microparticles, and
4) eventually removing the porous glass material containing the glass microparticles from the mold,
thereby obtaining the glass macroporous matrix.

"Microparticle" refers therein to a support having one of its dimensions comprised between 10 et 900 µm. The shape of the microparticle may be selected among spher, for example hollow spheres, oval, plate,fibers, flat disc, for example flat microstructure disc, biconcave disc or irregular. Micropoarticles all may have the same shape, or it may be a mixture of different shapes. Also, microparticles may all have the same size, or it may be a mixture of different sizes of microparticles. Advantageously, the size and the shape of the porosity of the matrix may be controled by choosing or controlling the physical features of the microparticles, as their size and/or dispersion in the mold. The microparticles may be hollow microparticles, i.e. comprising air, or they may be a solid, i.e. a full, material.

The glass microparticle may be porous or non porous. It can be for example Glass micro-sphere 3MTM-K20, or any other glass microparticle. It may be in bioactive glass.

In case where the glass microparticles are porous, it may be advantageous that they be hydrated, i.e. they contain water into their pores, as the water contained in the porosity may catalyze the gelling reaction during sol-gel bonding. In this purpose, the microparticles may be for example soaked in water to ensure that the water enters their pores before the pouring of the mold with the microparticles.

The mold used in the process of the invention may be a cavity comprising an entry allowing the introduction of the sol-gel solution. This entry may be sealed once the sol-gel solution is introduced. Advantageously, the mould may comprise a hole allowing to evacuate gases formed at the time of the stage of gelation and/or the stage of drying of the sol-gel solution.

In step 1), the glass microparticles may have, in the mold, the same shape, or different shapes. In the mold, the glass microparticles may have the same size, or may have different sizes. Advantageously, the arrangement of the microparticles in the mold creates spaces between the microparticles, therefore defining pores into the matrix. The size of pores may vary depending on the size of the microparticles.

In step 1, the loading of glass microparticles in the mold may be in the range of from 0.1 to 80.0%, for example of from 50.0% to 80.0%, or of from 60.0% to 75.0%, for example of from 60.0% to 74.0%. Preferably, the mold is filled to full capacity.

"Sol-gel solution" refers herein to a liquid solution that is able to produce a solid material by conversion of monomers into a colloidal solution (sol) that acts as the precursor for an integrated network (or gel) of either discrete particles or network polymers.

The sol-gel solution used in the first process of the invention may comprise a compound selected among silicon dioxide, bore oxide, aluminium oxide and titanium oxide. This sol-gel solution is able to form glass, eventually a bioactive glass, when it is submitted to gelation and drying. The sol-gel solution may therefore comprise at least about 50% silicon dioxide, or bore oxide, or aluminium oxide or titanium oxide. The sol-gel solution may therefore comprise of from 50% to 100% of silicon dioxide, for example of from 50% to 90%. Sol-gel solution may further comprise at least one mineral compound and/or at least one organic compound. The at least one mineral compound may be selected among hydroxyapatites, calcium, potassium, fluore, inorganic growth factors. The at least one organic compound may be selected among collagen, peptides, polysaccharides, polymeric materials, such as polystyrene, aliphatic polyesters such as polycarprolactone (e.g., poly(s-caprolactone)), poly(lactate), poly(glycolate), poly(dioxanone), polyhydroxyallanoates and their copolymers, polyolefin, gelatin, chitosan, zein, polyvinylpyrrolidone, hydroxypropyl methyl cellulose, polyethylene oxide, polyethylenimine polyvinyl alcohol, polyamides, or polyurethanes, organic growth factors, differentiation factors, cell adhesion molecules or proteins, pharmaceutical small molecules, absorbent particles, fluorophores, organic dyes, this list not being limitative. These compounds allow conferring specific properties to the sol-gel solution, and therefore to the matrix. For example, collagen may allow increasing cell adhesion and cell growth. For example, hydroxyapathite may promote osteogenesis.

Organic growth factors may be for example VEGF, collagen, bone morphogenic factorbeta, EGF, PDGF, NGF, FGF, IGF, or TGF.

Differentiation factors may be for example neurotrophin, CSF, or TGF.

Cell adhesion molecules or proteins may be for example a member of
immunoglobulin superfamily such as IgSF CAMs-the integrins, the cadherins, and the selectins.

Absorbent particles may be for example polyacrylamide copolymer, ethylene maleic anhydride copolymer, cross-linked carboxymethylcellulose, polyvinyl alcohol copolymers, cross-linked polyvinypyrrolidone, cross-linked polyethylene oxide, starch grafted copolymer of polyacrylonitrile, polyurethane, Pluronic®, gelatin, silica gel, cross-linked dextran (saphadex), Alginate, Agar-agar, microbial cellulose, modified clay, or their mixtures.The sol-gel solution has a composition allowing it to gelify. Advantageously, the sol-gel solution may gelify after drying. Advantageously, the drying period has just to be long enough so that the solution is dried. However, the drying period may be longer, without impairing physicochemical properties of the matrix. The man skilled in the art knows how to adapt the drying period. Advantageously, the drying step may be accelerated by heating and/or by vaccum, so as to facilitate the removal of solvants and water

Advantageously, the glass microparticles and the sol-gel solution have the same composition, so that the physicochemical and biological properties of the sol-gel solution are not modified.

In step 2) of the process of the invention, filing the mold may be realized by injection of the sol-gel solution in the mold, or by pouring the sol-gel solution in the mold. Thus, the spaces between the glass microparticles are totally occupied by the sol-gel solution. The filing of the mold may be continued until complete filling of it, or at least until complete overlapping of the glass microparticles with the sol-gel solution. This avoids that the sol-gel dries at this step of the process.

At this stage of the process, a cover may be placed at the top of the mold, in order to cover the sol-gel solution containing the glass microparticles. Advantageously, this may avoid drying of the sol-gel solution at this stage of the process.

In step 3), the gelification of the sol-gel solution may be realized by any mean known in this purpose by the man skilled in the art, as by using acidic conditions, for example chlorhydric acid, nitric acid, acetic acid, citric acid, or by using basic conditions, for example ammonia, (3-aminopropyl)triethoxysilane), or by using acidic conditions first and then basic conditions.

Then, the sol-gel material may be dried in order to remove any liquid from the material. Once the drying step is performed, the solid glass material is obtained.

During the drying step, the sol-gel solution material may retract, let blank in the gaps and concentrate in contact areas between the microparticles. After complete gelification and drying, the sol-gel used may link, as a glue, the microparticles with each other.

Advantageously, the gelling step and the drying step are performed at room temperature, i.e. at a temperature comprised between about 15 °C to about 25 °C.

Advantageously, the linkage is most effective if the porosity of the microparticles is high, so that the sol-gel solution penetrate into the porosities of the microparticles.

The glass gel formed at the end of this step works as a binder to link the glass microparticles with each other. The glass gel containing the glass microparticles is called the "glass material". The three-dimensional structure of the glass material provides a controllable and open cell culture system, in which glass microparticles are linked up with each other to form a rigid or relatively rigid lattice of dispersed microparticles.

This glass material may be porous, for example microporous or macroporous.

Then, in step 4), the glass material containing the microparticles may be removed from the mold.

A matrix obtained by implementing the process of the invention can be used for in vitro cell or tissue culture, particularly adherent cells. The cells can penetrate and proliferate comfortably into the matrix, especially in the gap between the glass microparticles, or may link to the surface of the microparticles and may multiply and spread.

The matrices obtained by implementing the process of the invention may also be used for preparing tissue substitutes, for example a spongy bone substitute. In this purpose, a bioactive glass tube is prepared which serves as a mold and as a matrix for regeneration of dense bone. This tube is filled with sol-gel beads, and the beads are then bonded together and to the mold by sol-gel pathway. In this embodiment, the porous glass material containing the glass microparticles is not removed from the mold. This spongy bone substitute is implantable to a human or animal subject.

In another aspect, the matrices obtained by implementing the process of the invention may be used for performing bioassay especially for studying the behavior of cells in a 3D matrix, for example after contacting them with a drug.

So, another object of the invention is a process of cell culturing comprising the steps of:
1) preparing a glass macroporous matrix by implementing the process of the invention,
2) applying cells at the surface of the matrix,
3) culturing the cells under culture conditions allowing the cells to colonize the matrix.

Step 2) may be performed by any suitable method known in the prior art. The cells may be for example in the form of a cell suspension.

Advantageously, any condition commonly used for culturing cells may be applied in step 3) of the process of cell culturing. It may be for example adding culture medium suitable for the cultured cells, adjusting atmospheric conditions, for example adding oxygen to the culture or adapting humidity of the culture.

A matrix of this invention can be used for in vitro cell or tissue culture, particularly cell culture that needs more cell spreading. When a cell suspension is administrated on the device, the agglomerates of cells can easily attach to the beads. The cells can penetrate and proliferate comfortably into the matrix. The cells can also spread randomly or orderly into the matrix depending on the size and shape of the microparticles.

The cells may be any cells, for example myofibroblastes, adipoblastes, chondroblastes, cémentoblastes, vodontoblastes, osteoblasts, neuroblasts, lymphocytes, tumoral cells, or stem cells.

This invention is further illustrated by the following examples with regard to the annexed drawings that should not be construed as limiting.

### Brief description of the drawings

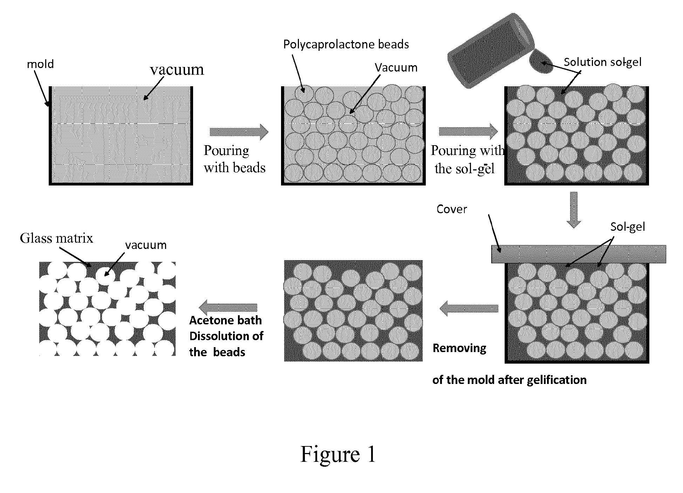

- La figure 1 represents the scheme of the process of the invention.

### EXAMPLES

### Exemple 1 : Preparation of a porous matrix by the first process of the invention

### Preparation of the beads :

1g of beads (Carroucell beads comprising 99.5% SiO₂, 0.15% collagen, 0.35% hydroxyapatite) of 160 µm in diameter in a sol-gel are poured into a 10ml beaker. 5 ml distilled water are then added. Sol-gel beads are mesoporous, they are soaked in water for 10 minutes to ensure that the water enters the pores of the beads. Excess water is removed and the beads are then slightly dried for 5 minutes in an oven at 60 ° C. Drying must be only partial, as the water contained in the porosity catalyzes the gelling reaction during sol-gel bonding.

### Pouring of the mold:

A container (mold) of 5 mm diameter and 3mm height is filled with the hydrated beads using a spatula.

### Preparation of the sol-gel solution:

Solution 1: 20 mg of hydroxyapatite (HAP) are dissolved in 2 ml of water in which 50 µl of HCl 34% are added. HCl solubilizes hydroxyapatite. After stirring the hydroxyapatite is completely dissolved.
Solution 2: In a pillbox in which 2 ml of TMOS (tetramethyl orthosilcate) are prélablement poured, 240µl solution 1 are added. 40 µl of type I collagen solution are then added under agitation. The hydrolysis of TMOS is exothermic. After cooling the mixture to room temperature, 1.2ml APTES (3-aminopropyl) triethoxysilane) are added to the solution. This solution can not gellify because the amount of water added is insufficient. Gelling will occur in contact with the previously moistened sol-gel beads.

### Addition of the sol-gel solution to the beads

120 µl of the previously prepared sol-gel solution is added into the mold. This solution occupies the free space between the beads. It gelled in contact with water trapped in mesoporosities of the beads that have been previously hydrated. Gelling is almost instant.

### Drying :

The whole is then dried at room temperature for 24h.

### List of references

**1.** WO2014197845.
**2.** WO201345671.

## Claims

1. A process for producing a glass macroporous matrix, comprising the steps of :
1) filing a mold with glass microparticles,
2) filing the mold obtained in step 1) with a sol-gel solution comprising a compound selected among silicon dioxide, bore oxide, aluminium oxide and titanium oxide, thereby covering the glass microparticles,
3) gelling and drying at room temperature the sol-gel solution, thereby obtaining a glass material containing the glass microparticles, and
4) eventually removing the porous glass material containing the glass microparticles from the mold,
thereby obtaining the glass macroporous matrix.

2. Process according to claim 1, wherein said sol-gel solution comprises at least one mineral compound and/or at least one organic compound.

3. Process according to claim 2, wherein said at least one mineral compound is selected among hydroxyapatites, calcium, potassium, fluore and inorganic growth factors.

4. Process according to claim 2, wherein said at least one organic compound is selected among collagen, peptides, polysaccharides, polymeric materials, such as polystyrene, aliphatic polyesters, polyolefin, polysaccharides, collagen, gelatin, chitosan, zein, polyvinylpyrrolidone, hydroxypropyl methyl cellulose, polyethylene oxide, polyethylenimine polyvinyl alcohol, polyamides, or polyurethanes, organic growth factors, differentiation factors, cell adhesion molecules or proteins, pharmaceutical small molecules, absorbent particles, fluorophores and organic dyes.

5. Process according to anyone of the preceding claims, wherein the shape of said glass microparticles is selected among sphere, oval, plate,fibers, flat disc, biconcave disc or irregular shape.

6. Process according to anyone of the preceding claims, wherein said glass microparticles used in step 1) are porous glass microparticles.

7. Process according to claim 6, wherein said porous glass microparticles are hydrated.

8. A process of cell culturing comprising the steps of:
1) preparing a glass macroporous matrix by a process as defined in anyone of the preceding claims,
2) applying cells at the surface of the matrix,
3) culturing the cells under culture conditions allowing the cells to colonize the matrix.

9. A process according to claim 8, wherein said cell is selected among myofibroblastes, adipoblastes, chondroblastes, cémentoblastes, vodontoblastes, osteoblasts, neuroblasts, lymphocytes, tumoral cells and stem cells.
